(19) Europäisches Patentamt
European Patent Office
Office européen des brevets

(11) Veröffentlichungsnummer: **0 373 491**
**A1**

# (12) EUROPÄISCHE PATENTANMELDUNG

(21) Anmeldenummer: **89122455.2**

(22) Anmeldetag: **06.12.89**

(51) Int. Cl.$^5$: **C07C 231/10, C07C 233/36, A61K 7/00, C11D 1/10**

(30) Priorität: **15.12.88 ES 8803808**

(43) Veröffentlichungstag der Anmeldung:
**20.06.90 Patentblatt 90/25**

(84) Benannte Vertragsstaaten:
**GR**

(71) Anmelder: **Henkel Kommanditgesellschaft auf Aktien**
**Henkelstrasse 67**
**D-4000 Düsseldorf 13(DE)**

(72) Erfinder: **Trius, Antonio D.**
**Puerto de la Orquidea, 75-77**
**E-Valldoreix Sant Cugat(ES)**
Erfinder: **Humbert, Miquel**
**Ferran Casablancas, 88**
**E-08902 Sabadell(ES)**
Erfinder: **Cuadrado, Francisco**
**Caspe, 141 esc. A 1,2**
**E-08013 Barcelona(ES)**
Erfinder: **Bigorra, Joaquin**
**Calassanc Duran, 41 esc. E 4,1**
**E-08203 Sabadell(ES)**
Erfinder: **Pomares, Jésus**
**Mejas Lequerica, 42, 2,4**
**E-08028 Barcelona(ES)**
Erfinder: **Ploog, Uwe**
**Haydnstrasse 6**
**D-5657 Haan(DE)**
Erfinder: **Uphues, Günter**
**Robert-Koch-Strasse 45**
**D-4019 Monheim(DE)**

## (54) Verfahren zur Herstellung von amphoteren grenzflächenaktiven Imidazolinderivaten.

(57) Bei der Herstellung von Gemischen amphoterer grenzflächenaktiver Imidazolinderivate, die hauptsächlich Verbindungen der Formel I enthalten,

$$R_1 - \overset{O}{\overset{\|}{C}} - NH - CH_2 - CH_2 - \underset{B}{\underset{|}{N}} - D \qquad (I)$$

in der $R_1$ einen $C_{5-21}$-Alkylrest, B einen $C_{2-4}$-Aminoalkyl- oder Hydroxyalkylrest und D einen $-R_2$-COOM-Rest bedeuten, wobei $R_2$ für einen $C_{1-4}$-Alkylenrest oder einen $C_{2-4}$-Alkenylenrest und M für H, $NH_4$ oder Alkalimetall steht, wird

A) ein Imidazolin der Formel II,

EP 0 373 491 A1

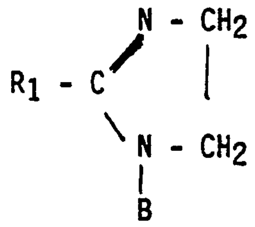

(II)

mit Wasser im Molverhältnis 1 : 8 bis 1 : 20 bei 50 bis 130° C unter atmosphärischem Druck oder geringem Überdruck in 1 bis 24 Stunden zu einem Gemisch von Amidoaminen hydrolysiert,
das erhaltene Amidoamingemisch mit einem Alkylierungsmittel der Formel X - D, in der X für Halogen oder Wasserstoffatom steht und D die oben angegebene Bedeutung hat, im Molverhältnis von Alkylierungsmittel zu Amidoaminen von 1 : 1 bis 4 : 1 mit Hilfe einer Base bei 40 bis 70° C bei pH 10 bis 11,5, gemessen in einer 10 gew.-%igen wässrigen Lösung des Reaktionsgemisches, alkyliert, und
C) das überschüssige Alkylierungsmittel bei 80 bis 95° C und pH 10 bis 11,5, gemessen in einer 10 gew.-%igen wässrigen Lösung des Reaktionsgemisches, hydrolysiert.

## Verfahren zur Herstellung von amphoteren grenzflächenaktiven Imidazolinderivaten

Die Erfindung betrifft ein neues Verfahren zur Herstellung von amphoteren Tensidprodukten der Formel I

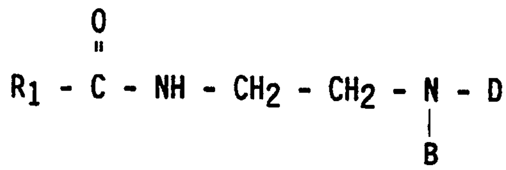

(I)

in der $R_1$ einen Alkylrest mit 5 bis 21 Kohlenstoffatomen, B einen Aminoalkyl- oder Hydroxyalkylrest mit 2 bis 4 Kohlenstoffatomen und D einen - $R_2$ - COOM - Rest bedeuten, wobei $R_2$ für eine Alkylenrest mit 1 bis 4 Kohlenstoffatomen oder einen Alkenylenrest mit 2 bis 4 Kohlenstoffatomen und M für Wasserstoff, Ammonium oder ein Alkalimetall steht. Diese Tenside ermöglichen im Gemisch mit anionischen Tensiden die Herstellung von Fertigformulierungen für die persönliche Pflege und Hygiene mit sehr geringer hautreizender Wirkung. Mit geringen Mengen herkömmlicher Verdikkungsmittel ermöglichen sie ohne weiteres die Einstellung der Viskosität, die für handelsübliche Fertigprodukte dieser Art erwünscht ist.

Das Verfahren besteht im wesentlichen aus der Hydrolyse eines Imidazolins der Formel II

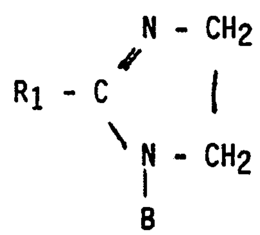

(II)

in der $R_1$ und B die oben angegebene Bedeutung haben, unter kontrollierten Bedingungen zu einem intermediären Amidoamin der Formel III,

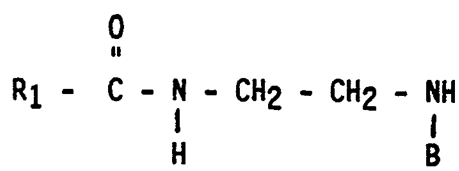

(III)

in der $R_1$ und B die oben angegebene Bedeutung haben, und der anschließenden Alkylierung dieses Amidoamins mit einem Alkylierungsmittel der Formel V,

X - D (IV)

in der X für ein Halogenatom oder ein Wasserstoffatom steht und D die oben angegebene Bedeutung hat, unter Zugabe einer Base zur Verschiebung des Alkylierungsgleichgewichtes in die gewünschte Richtung durch Neutralisation der entstehenden Halogenwasserstoffssäure. Bei dieser Base kann es sich um Ammoniumhydroxid oder um ein beliebiges Alkalihydroxid, vorzugsweise Natriumhydroxid, handeln. All das zielt in erster Linie auf die Bildung der Endverbindung der Formel I ab.

Bei den Imidazolinverbindungen der Formel II, die als Ausgangsmaterial für das erfindungsgemäße Verfahren in Betracht kommen, handelt es sich um bekannte Stoffe, die nach bekannten Verfahren der organischen Synthese, beispielsweise gemäß der EP-PS 2943 und der ES-PS 540 640 durch Reaktion einer Fettsäure der Formel $R_1$-COOH oder eines Fettsäuremethylesters der Formel $R_1$ - $COOCH_3$ mit einem Diamin der Formel $H_2N$ - $CH_2$ - $CH_2$ - NH - B hergestellt werden können.

Diese Fettsäuren und die Fettsäurereste der in Frage kommenden Methylester können natürlicher oder synthetischer Herkunft sein. Als Ausgangsmaterial kommen beispielsweise Capron-, Heptan-, Capryl-, Undecyl-, Laurin-, Myristin-, Palmitin-, Stearin-, Behen-, Arachin-, Öl-, Linol-, Ricinol-, Ethylhexan-, und

Isostearinsäure sowie deren Ester in betracht. Dabei können die einzelne Fettsäuren oder Ester einzelner Fettsäuren für die Synthese der Imidazoline verwendet werden. Es können aber auch Fettsäure-und Fettsäureestergemische eingesetzt werden, wie sie durch Fettspaltung oder Umesterung mit Methanol direkt aus natürlich vorkommenden Fetten und Ölen beispielsweise aus Kokosöl, erhältlich sind.

Als Alkylierungsmittel der Formel V kommen insbesondere Halogenalkansäuren (X = Halogen; $R_2$ = Alkylenrest mit 1 bis 4 Kohlenstoffatomen), vorzugsweise Monochloressigsäure, und Alkensäuren (X = Wasserstoff; $R_2$ = Alkenylrest mit 2 bis 4 Kohlenstoffatomen), vorzugsweise Acrylsäure, und deren Salze, insbesondere deren Alkalisalze, in Betracht. Natriummonochloracetat ist als Alkylierungsmittel besonders bevorzugt.

Bei den Diaminen der oben angegebenen Formel handelt es sich um solche, in denen B für einen Hydroxyalkyl- oder Aminoalkylrest steht, wobei B vorzugsweise einen Hydroxyalkylrest mit 2 bis 4 Kohlenstoffatomen, insbesondere einen Hydroxyethylrest bedeutet.

Bei den in der EP-PS 1006 und der ES-PS 540 508 beschriebenen Verfahren zur Herstellung von amphoteren Tensiden der hier zu betrachtenden Art wird ein Imidazolin der Formel II direkt mit einer wässrigen Lösung eines Alkylierungsmittels (V) mittels einer Base zur Reaktion gebracht. In diesen Verfahren wird gleichzeitig die Hydrolyse des Imidazolins und seine Alkylierung bewirkt. Es entsteht ein Gemisch aus Mono- und Dialkylamidoaminen und im allgemeinen wird ein Produkt mit niederer Viskosität erhalten. Wie bereits oben erwähnt, wird das Imidazolin schon zum Teil alkyliert, bevor durch seine Hydrolyse ein Gemisch aus Amidoaminen mit linearen Ketten entsteht, an denen die Alkylierung dann weitergeht.

Das Endprodukt besteht aus einer komplexen Mischung von alkylierten Verbindungen, die zum Teil durch Alkylierung des Imidazolins, anschließende hydrolytische Spaltung und weitere Alkylierung der linearen Hydrolyseprodukte, zum anderen Teil durch direkte Hydrolyse des Imidazolins und Alkylierung der linearen Amidoamine entstanden sind. Die Zusammensetzung des Endproduktes hängt von den Temperatur- und pH-Bedingungen ab, unter denen die Zugabe sowohl des Imidazolins als auch des Alkalihydroxids erfolgt. Arbeitet man bei einem hohen pH-Wert, so hat die Hydrolyse des Imidazolins den Vorrang vor der direkten Alkylierung und es entstehen Produkte der Formel I

$$R_1 - \overset{\overset{O}{\|}}{C} - NH - CH_2 - CH_2 - \underset{\underset{B}{|}}{N} - D \qquad (I)$$

in der B und D die oben angegebene Bedeutung haben. Beim Arbeiten im technischen Maßstab ist es jedoch sehr schwierig, unter den vorgenannten Bedingungen auf Dauer Endprodukte zu erhalten, deren Viskositäten sich innerhalb eines akzeptablen engen Schwankungsbereichs bewegen.

Arbeitet man andererseits bei pH-Werten in der Nähe des Neutralpunktes oder im mäßig alkalischen Bereich, beispielsweise bei pH 7 bis 11, so ist die Geschwindigkeit der Alkylierung des Imidazolins größer als die seiner Hydrolyse. Es entsteht ein hochalkyliertes Endprodukt, das eine niedere Viskosität aufweist und im wesentlichen aus einer Mischung von Verbindungen der Formeln VI bis VIII besteht,

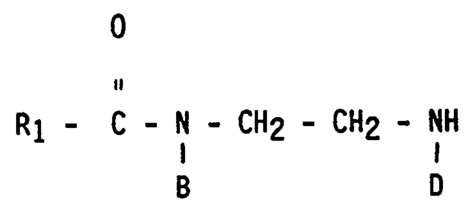

(VI)

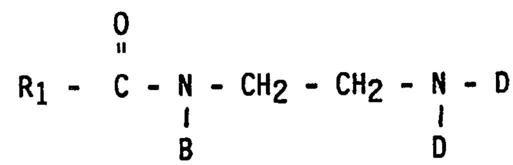

(VII)

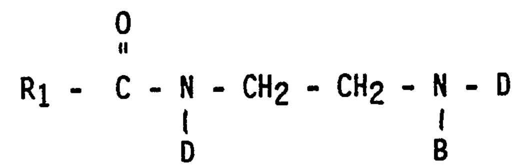

(VIII)

in denen $R_1$, B und D wieder die oben angegebene Bedeutung haben. Nach dem Ende der Reaktion muß ein gegebenenfalls vorhandener Überschuß an Alkylierungsmittel so hydrolysiert werden, daß bei der Verwendung des Endproduktes keine unerwünschten Effekte, beispielsweise starke Hautreizung, auftreten. Wenn beispielsweise Natiummonochloracetat als Alkylierungsmittel eingesetzt wir, entsteht bei dessen Hydrolyse Natriumchlorid und Natriumglycolat. Bei diesem Hydrolyseprozeß werden auch die tertiären Amide der Formeln (VI) bis (VIII) angegriffen, wobei unerwünschte Alkaliseifen entstehen, die im Endprodukt zu einer Erhöhung der Viskosität führen und, noch schwerwiegender, die Verträglichkeit des Produktes mit hartem Wasser stark beeinträchtigen.

Ein weiterer Nachteil der auf diesem Wege hergestellten Produkte besteht darin, daß sie große Mengen Verdickungsmittel, beispielsweise Polyethylenglycoldistearat 6000, erfordern, wenn sie zusammen mit Alkylsulfaten der Formel IX oder Alkylethersulfaten der Formel X

$R_2 - O - SO_3 - M$ (IX)

$R_2 - (OCH_2CH_2)_n - O - SO_3M$ (X)

in denen $R_2$ einen Alkylrest mit 10 bis 18 Kohlenstoffatomen, n eine Zahl von 1 bis 10 und M ein Alkalimetall, vorzugsweise Natrium bedeutet, formuliert werden, damit in den Fertigprodukten die Viskositäten erzielt werden, die der Markt für diese Erzeugnisse verlangt.

Faßt man die Nachteile des bisherigen Standes der Technik zusammen, so läßt sich feststellen, daß nach den bekannten Verfahren entweder Produkte mit hoher aber schlecht reproduzierbarer Viskosität erhalten werden, oder Produkte mit niederer Viskosität, die einerseits bei der praktischen Formulierung erhebliche Mengen an Verdickungsmitteln erfordern, um in den Fertigprodukten ausreichende Viskositätswerte zu erzielen, und die andererseits durch ihren erheblichen Gehalt an Alkalimetallseifen zu einer Stabilitätseinbuße gegenüber hartem Wasser führen.

Gegenstand der Erfindung ist ein Verfahren zur Herstellung von Gemischen amphoterer grenzflächenaktiver Imidazolinderivate hoher bis mittlerer Viskosität, die hauptsächlich Verbindungen der Formel I enthalten,

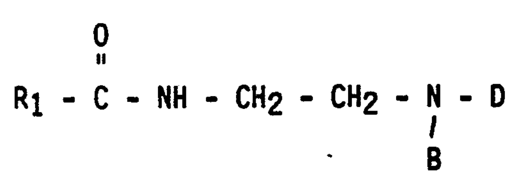

(I)

in der $R_1$ einen Alkylrest mit 5 bis 21 Kohlenstoffatomen, B einen Aminoalkyl- oder Hydroxyalkylrest mit 2 bis 4 Kohlenstoffatomen, vorzugsweise einen Hydroxyethylrest, und D einen $-R_2$-COOM-Rest bedeuten, wobei $R_2$ für einen Alkylenrest mit 1 bis 4 Kohlenstoffatomen oder einen Alkenylenrest mit 2 bis 4 Kohlenstoffatomen und M für Wasserstoff, Ammonium oder ein Alkalimetall, vorzugsweise Natrium, steht. Das neue Verfahren ist dadurch gekennzeichnet, daß man

A) ein Imidazolin der Formel II,

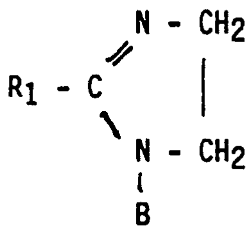

(II)

in der $R_1$ und B die oben angegebene Bedeutung haben, mit Wasser in einem Molverhältnis von 1 : 8 bis 1 : 20 bei Temperaturen zwischen 50 und 130° C unter atmosphärischem Druck oder geringem Überdruck im Verlauf von 1 bis 24 Stunden zu einem Gemisch von Amidoaminen der Formeln III und IV

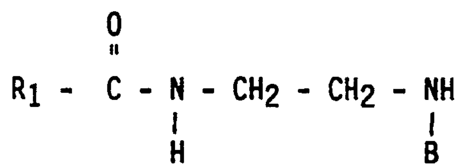

(III)

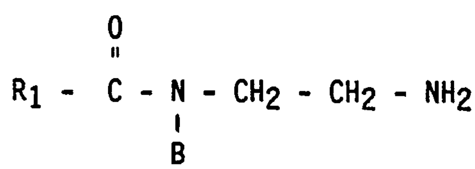

(IV)

in denen $R_1$ und B die oben angegebene Bedeutung haben, hydrolysiert,

B) das Gemisch der erhaltenen Amidoamine mit einem Alkylierungsmittel der formel V

X - D (V)

in der X für ein Halogenatom, vorzugsweise ein Chloratom, oder ein Wasserstoffatom steht und D die oben angegebene Bedeutung hat, in einem Molverhältnis von Alkylierungsmittel (V) zu Amidoaminen (III + IV) zwischen 1,5 : 1 und 2,5 : 1 mit Hilfe einer Base bei 40 bis 70° C unter Einhaltung eines pH-Wertes zwischen 10 und 11,5, gemessen in einer 10 gew.-%igen wässrigen Lösung des Reaktionsgemisches, alkyliert, so daß in erster Linie die Endverbindung der Formel I und in geringerem Maße auch Verbindungen der Formeln VI und VII entstehen,

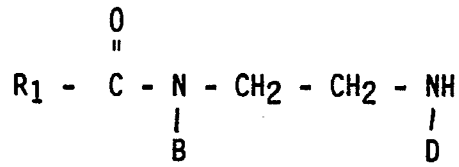

(VI)

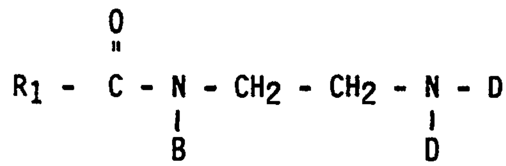

(VII)

in denen $R_1$, und D die oben angegebene Bedeutung haben, und anschließend

C) das überschüssige Alkylierungsmittel der Formel V bei einer Temperatur zwischen 80 und 90° C unter Einhaltung eines pH-Wertes zwischen 10 und 11,5, gemessen in einer 10 gew.-%igen wässrigen Lösung des Reaktionsgemisches, hydrolysiert.

Die mit Hilfe des erfindungsgemäßen Verfahrens hergestellten Produkte weisen von sich aus eine hohe Viskosität auf, wodurch die anwendungstechnischen Nachteile, mit denen die Produkte des Standes der Technik behaftet sind, in erheblichem Maß reduziert werden. Mit Hilfe des neuen Verfahrens können Produkte mit hohen, jedoch nicht übermäßigen Viskositäten im Bereich von 1000 bis 5000 cP hergestellt werden. Diese Produkte können ohne weiteres in den industrieüblichen Anlagen gehandhabt und gefördert werden. Weiterhin kann die Menge des Verdickungsmittels, das erforderlich ist, um die vom Markt geforderten Viskositätswerte bei Fertigprodukten, beispielsweise Shampoos, einzustellen, erheblich reduziert werden. Schließlich ist der Gehalt an störenden Metallseifen in den nach dem erfindungsgemäßen Verfahren erhaltenen Produkten wesentlich geringer als in den nach den bekannten Verfahren hergestellten Produkten.

Überraschender Weise wird durch die gezielte Hydrolyse des Imidazolins vor der Alkylierung erreicht, daß die Zusammensetzung des Endprodukts kontrolliert und so ausgerichtet werden kann, daß die oben erwähnten Eigenschaften erzielt werden.

Die Herstellung des intermediären Imidazolins der Formel II

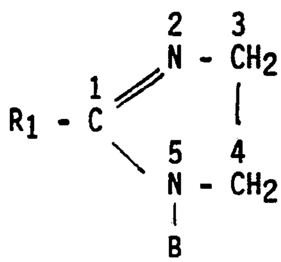

(II)

in der $R_1$ und $B_1$ die oben angegebene Bedeutung haben, ist hinreichend bekannt und beispielsweise in der ES-PS 540 640 und der EP-PS 2943 beschrieben.

Die Hydrolyse des Imidazolins der Formel II kann zwei verschiedene Amidoamintypen hervorbringen. Das Amidoamin der Formel III

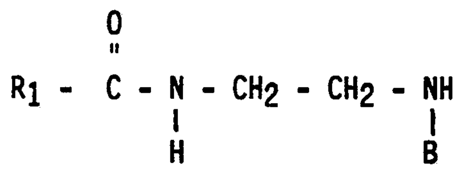

(III)

entsteht, wenn die Hydrolysespaltung zwischen dem Kohlenstoffatom 1 und dem Stickstoffatom 5 des Imidazolinringes eintritt. Das Amidoamin der Formel IV

$$R_1 - \overset{\overset{\large O}{\|}}{C} - \underset{\underset{\large B}{|}}{N} - CH_2 - CH_2 - NH_2 \qquad (IV)$$

entsteht, wenn die Hydrolysespaltung zwischen dem Kohlenstoffatom 1 und dem Stickstoffatom 2 des Imidazolinringes eintritt.

Es wurde überraschenderweise festgestellt, daß bei der Hydrolyse des Imidazolins eine totale Spaltung der -C = N-Bindung abläuft und zwar nach einem kinetischen Mechanismus, in dem die Bildung des Amidoamins der Formel IV bevorzugt ist. Daneben läuft ein langsamer Isomerisierungsprozeß ab, bei dem das Amidoamin der Formel IV unter Bildung des Amidoamins der Formel III verschwindet, welch letzteres thermodynamisch stabiler ist. Es ist deshalb bei der Hydrolyse möglich, durch Steuerung der Temperatur- und Zeitbedingungen das gewünschte Verhältnis zwischen den beiden Amidoamintypen (III) und (IV) im Hydrolyseprodukt einzustellen. Die Eigenschaften des Endproduktes sind optimal, wenn in der Hydrolyse des Imidazolins ein Anteil an Amidoaminen der Formel III von 50 bis 95 Gew.-% vorzugsweise von 80 bis 90 Gew.-%, bezogen auf das gesamte Hydrolyseprodukt, erreicht wird.

Wenn es möglich wird, die Zusammensetzung des intermediären Hydrolyseproduktes zu steuern und festzulegen, dann kann man damit auch ein vollkommen definiertes Endprodukt erhalten. Im konkreten Fall werden bei der Alkylierung mit einem Alkylierungsmittel der Formel X-D aus dem Amidoamin der Formel IV die Verbindungen der Formeln VI und VII erhalten. Die Alkylierung des Amidoamins der Formel III hingegen führt zu dem Alkylierungsprodukt der Formel I, das im allgemeinen den größten Teil des Endproduktes ausmacht.

Die Hydrolyse des Imidazolins der Formel II kann bei atmosphärischem Druck oder leichtem Überdruck in einem Temperaturbereich zwischen 50 und 130 °C, vorzugsweise zwischen 80 und 90 °C und mit einem Molverhältnis von Imidazolin zu Wasser zwischen 1 : 8 bis 1 : 20, vorzugsweise zwischen 1 : 8 bis 1 : 15 durchgeführt werden. Dabei ist zu berücksichtigen, daß die Bildung des sekundären Amidoamins der Formel III zu Lasten des tertiären Amidoamins der Formel IV durch lange Hydrolysezeiten, insbesondere Hydrolysezeiten zwischen 1 und 24 Stunden, vorzugsweise zwischen 6 und 15 Stunden, und hohe Temperaturen, insbesondere Temperaturen zwischen 50 und 130 °C, vorzugsweise zwischen 90 und 110 °C, begünstigt wird.

Die Alkylierung der Hydrolyseprodukte erfolgt wie oben bereits angegeben nach bekannten Verfahren. Dabei wird das in der vorangegangenen Hydrolysestufe erhaltene Gemisch der Amidoamine einer wäßrigen Lösung des Alkylierungsmittels, vorzugsweise Natriummonochloracetat, bei einer Temperatur zwischen 40 und 70 °C, vorzugsweise zwischen 45 und 55 °C, zugesetzt. Das Molverhältnis von Amidoaminen zu Alkylierungsmitteln liegt dabei zwischen 1 : 1 und 4 : 1, vorzugsweise zwischen 1,5 : 1 und 2,8 : 1. Anschließend wird allmählich eine wässrige Alkalihydroxidlösung, vorzugsweise Natriumhydroxidlösung, unter Beibehaltung der angegebenen Temperaturwerte mit einer solchen Geschwindigkeit zugegeben, daß der pH-Wert, gemessen in einer 10 gew.-%igen Lösung des Reaktionsgemisches, zwischen 10 und 11,5, vorzugsweise zwischen 10,8 und 11,2, gehalten wird und die Alkylierung den vorhandenen Aminstickstoffatomen in einem angemessenen Zeitraum von etwa 3 bis 6 Stunden stattfindet. Die genannten Bedingungen schaffen gleichzeitig die Möglichkeit, in dieser Phase des Prozesses die Geschwindigkeit der Hydrolyse des Alkylierungsmittels entsprechend der Gleichung

$$ClCH_2COOONa + NaOH \rightarrow NaCl + HOCH_2COONa$$

auf einen niedrigen Wert einzustellen. Wenn der gewünschte Alkylierungsgrad erreicht ist, wird die Temperatur des Reaktionsgemisches auf 80 bis 95 °C, vorzugsweise auf 85 bis 90 °C, erhöht. Das Reaktionsgemisch wird dann unter laufender Zugabe von Natriumhydroxidlösung zur Aufrechterhaltung eines pH-Wertes innerhalb der oben angegebenen Grenzen so lange auf dieser Temperatur gehalten, bis das überschüssige Alkylierungsmittel zerstört ist.

## Beispiele

### Beispiel 1

In einem mit Heizung und mechanischer Rührvorrichtung versehenen Labor-Glasreaktionsgefäß wurden 165,6 g (0,62 Mol) Hydroxyethylalkylimidazolin ($R_1$ = $C_{11}$; über 95 Gew.-% Imidazolingehalt) vorgelegt und unter Rühren auf 80 °C erwärmt. In einem Zeitraum von einer halben Stunde wurden 128 g (7,1 Mol) destilliertes Wasser zudosiert. Die Mischung wurde unter Rühren 15 Stunden lang zum Rückflußkochen erhitzt, so daß durch Hydrolyse eine wässrige Lösung eines Gemisches von Amidoaminen erhalten wurde, in dem bezogen auf Trockensubstanz, 83 Gew.-% sekundäres Amidoamin (III) enthalten waren.

In einem zweiten Labor-Glasreaktionsgefäß, das ebenso ausgestattet war wie das vorgenannte, wurden 143,6 g (1,23 Mol) Natriummonochloracetat in 482,5 g destilliertem Wasser gelöst. Diese Lösung wurde auf 50 °C erwärmt. Danach wurde unter Rühren innerhalb einer halben Stunde die oben beschriebene Mischung aus Amidoaminen zudosiert. Anschließend wurde 3 Stunden lang unter Beibehaltung der Temperatur von 50 °C und unter weiterem Rühren 64 g einer 50 gew.-%igen wässrigen Natriumhydroxydlösung so zudosiert, daß der pH-Wert einer 10 gew.-%igen wässrigen Lösung des Reaktionsgemisches zwischen 11,1 und 11,5 lag. Schließlich wurde die Temperatur auf 90 °C erhöht und im Verlauf von 5 Stunden 16 g 50 gew.-%ige Natriumhydroxidlösung zudosiert, so daß der pH-Wert einer 10 gew.-%igen wässrigen Lösung des Reaktionsgemisches zwischen 10,0 und 10,5 lag. Das Endprodukt hatte einen Wassergehalt von 65 Gew.-% und bei 20 °C eine Viskosität von 1500 cP, gemessen mit einem Brookfield-Viskosimeter.

Beispiel 2

In das im Beispiel 1 beschriebene Labor-Glasreaktionsgefäß wurden 133 g (0,5 Mol) Hydroxyethylalkylimidazolin ($R_1$ = $C_{11}$; über 95 Gew.-% Imidazolingehalt) vorgelegt und auf 105 °C erwärmt. Unter Rühren wurden innerhalb einer halben Stunde 103 g (5,7 Mol) destilliertes Wasser zudosiert. Die Mischung wurde unter Rühren 2 Stunden lang zum Rückflußkochen erhitzt, so daß eine wässrige Lösung eines Gemisches von Amidoaminen erhalten wurde, in dem, bezogen auf Trockensubstanz, 73 Gew.-% Amidoamin (III) enthalten waren.

Die Alkylierung erfolgte unter den gleichen Bedingungen, wie sie im Beispiel 1 beschrieben sind, dabei wurden 155,8 g (1,3 Mol) Natriummonochloracetat, 107 g 50 gew.-%ige wässrige Natriumhydroxidlösung und 502 g destilliertes Wasser eingesetzt.

Das Endprodukt hatte einen Wassergehalt von 65 Gew.-% und bei 20 °C eine Viskosität von 180 cP, bestimmt mit einem Brookfield-Viskosimeter.

Beispiel 3

Mit den gleichen Reagentien und Mengen wie in Beispiel 2, jedoch unter Durchführung der Hydrolyse bei 105 °C im Verlauf von 6 Stunden, wurde eine wäßrige Lösung von Amidoaminen mit einem Gehalt an 96 Gew.-% sekundärem Amidoamin (III), bezogen auf Trokkensubstanz, erhalten.

Unter Verwendung der gleichen Reagentien in den gleichen Mengen und unter Einhaltung der gleichen Bedingungen wie im Beispiel 2 wurde ein Endprodukt mit einem Wassergehalt von 65 Gew.-% und einer Viskosität bei 20 °C von 5800 cP, gemessen mit einem Brookfield-Viskosimeter, erhalten.

Beispiel 4

Mit den gleichen Reagentien in gleichen Mengen und unter den gleichen Bedingungen wie im Beispiel 1, jedoch unter Verwendung eines Alkylhydroxyethylimidiazolins der Formel I, dessen Substituenten $R_1$ aus einem Gemisch von Alkylresten mit 7 bis 17 Kohlenstoffatomen, abgeleitet aus dem Fettsäureanteil des Kokosöl, wurde ein Endprodukt mit einem Wassergehalt von 65 Gew.-% und einer Viskosität bei 20 °C von 1200 cP, bestimmt mit einem Brookfield-Viskosimeter, erhalten.

**Ansprüche**

1. Verfahren zur Herstellung von Gemischen amphoterer grenzflächenaktiver Imidazolinderivate hoher bis mittlerer Viskosität, die hauptsächlich Verbindungen der Formel I enthalten,

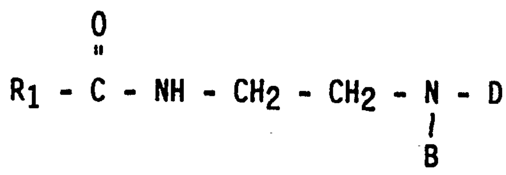

(I)

in der $R_1$ einen Alkylrest mit 5 bis 21 Kohlenstoffatomen, B einen Aminoalkyl- oder Hydroxyalkylrest mit 2 bis 4 Kohlenstoffatomen, vorzugsweise einen Hydroxyethylrest, und D einen $-R_2$-COOM-Rest bedeuten, wobei $R_2$ für eine Alkylenrest mit 1 bis 4 Kohlenstoffatomen oder einen Alkenylenrest mit 2 bis 4 Kohlenstoffatomen und M für Wasserstoff, Ammonium oder ein Alkalimetall, vorzugsweise Natrium, steht, dadurch gekennzeichnet, daß man

A) ein Imidazolin der Formel II,

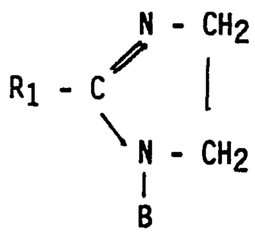

(II)

in der $R_1$ und B die oben angegebene Bedeutung haben, mit Wasser in einem Molverhältnis von 1 : 8 bis 1 : 20 bei Temperaturen zwischen 50 und 130 °C unter atmosphärischem Druck oder geringem Überdruck im Verlauf von 1 bis 24 Stunden zu einem Gemisch von Amidoaminen der Formeln III und IV

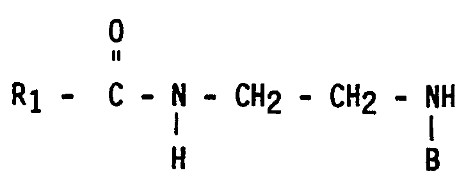

(III)

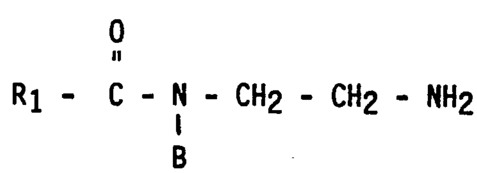

(IV)

in denen $R_1$ und B die oben angegebene Bedeutung haben, hydrolysiert,

B) das Gemisch der erhaltenen Amidoamine mit einem Alkylierungsmittel der Formel V,

X - D (V)

in der X für ein Halogenatom, vorzugsweise ein Chloratom oder ein Wasserstoffatom, steht und D die oben angegebene Bedeutung hat, in einem Molverhältnis von Alkylierungsmittel (V) zu Amidoaminen (III + IV) zwischen 1 : 1 und 4 : 1 mit Hilfe einer Base bei 40 bis 70 °C unter Einhaltung eines pH-Wertes zwischen 10 und 11,5, gemessen in einer 10 gew.-%igen wässrigen Lösung des Reaktionsgemisches, alkyliert, so daß in erster Linie die Endverbindung der Formel I und in geringerem Maße auch Verbindungen der Formeln VI und VII entstehen,

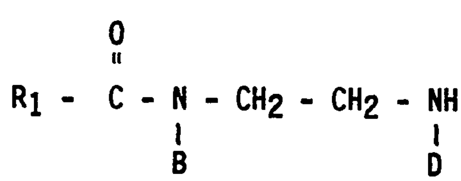

(VI)

```
              O
              "
R1 -  C - N - CH2 - CH2 - N - D          (VII)
          |               |
          B               D
```

in denen $R_1$, und D die oben angegebene Bedeutung haben, und anschließend.

C) das überschüssige Alkylierungsmittel der Formel V bei einer Temperatur zwischen 80 und 95 °C unter Einhaltung eines pH-Wertes zwischen 10 und 11,5, gemessen in einer 10 gew.-%igen wässrigen Lösung des Reaktionsgemisches, hydrolysiert.

2. Verfahren nach Anspruch 1, dadurch gekennzeichnet, daß in den Formeln I, V, VI und VII $R_2$ in D einen Ethylenrest und vorzugsweise einen Methylenrest bedeutet.

3. Verfahren nach den Ansprüchen 1 und 2, dadurch gekennzeichnet, daß bei der Hydrolyse das Molverhältnis von Imidazolin (II) zu Wasser zwischen 1 : 8 bis 1 : 15 liegt.

4. Verfahren nach den Ansprüchen 1 bis 3, dadurch gekennzeichnet, daß die Hydrolyse des Imidazolins (II) bei einer Temperatur zwischen 90 und 110 °C durchgeführt wird.

5.Verfahren nach den Ansprüchen 1 bis 4, dadurch gekennzeichnet, daß die Hydrolyse des Imidazolins (II) im Verlauf von 6 bis 15 Stunden durchgeführt wird.

6. Verfahren nach den Ansprüchen 1 bis 5, dadurch gekennzeichnet, daß die Alkylierung bei einer Temperatur zwischen 45 und 55 °C und bei einem pH-Wert zwischen 10,8 und 11,2, gemessen in einer 10 gew.-%igen Lösung des Reaktionsgemisches, durchgeführt.

7. Verfahren nach den Ansprüchen 1 bis 6, dadurch gekennzeichnet, daß bei der Alkylierung das Molverhältnis von Amidoaminen zu Alkylierungsmittel zwischen 21,5 : 1 und 2,8 : 1 liegt.

8. Verfahren nach den Ansprüchen 1 bis 7, dadurch gekennzeichnet, daß die Hydrolyse des überschüssigen Alkylierungsmittel (V) bei 85 bis 90 °C unter Einhaltung eines pH-Wertes zwischen 10,8 und 11,2, gemessen in einer 10 gew.-%igen wässrigen Lösung des Reaktionsgemisches, durchgeführt wird.

Europäisches Patentamt

# EUROPÄISCHER RECHERCHENBERICHT

Nummer der Anmeldung

EP 89 12 2455

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 001 006 (ALBRIGHT & WILSON LTD)<br>* Patentansprüche *<br>--- | 1 | C 07 C 231/10<br>C 07 C 233/36<br>A 61 K 7/00<br>C 11 D 1/10 |
| A | DE-A-2 725 780 (THE MIRANOL CHEMICAL CO.)<br>* Patentansprüche; Seiten 10,11 *<br>----- | 1 | |
| | | | **RECHERCHIERTE SACHGEBIETE (Int. Cl.5)** |
| | | | C 07 C 231/00<br>C 07 C 233/00<br>C 07 D 233/00 |

Der vorliegende Recherchenbericht wurde für alle Patentansprüche erstellt

| Recherchenort | Abschlußdatum der Recherche | Prüfer |
|---|---|---|
| DEN HAAG | 02-03-1990 | SANCHEZ Y GARCIA J.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)